# EUROPEAN PATENT SPECIFICATION

(11) **EP 3 503 829 B1**
(45) Date of publication and mention of the grant of the patent: **14.10.2026**
(21) Application number: 17844516.9
(22) Date of filing: 25.08.2017
(51) Int. Cl.: A61B 17/68, A61B 34/00, A61B 90/50, B25B 1/04, B25B 3/00, B25B 5/04, B25B 5/10, B25J 15/04, B25J 18/04, H01R 13/00, H01R 13/24, H01R 24/86, A61B 17/00, A61B 17/29, H01R 107/00

(54) **QUICK-RELEASE TOOL COUPLER AND RELATED SYSTEMS**
SCHNELL LÖSBARE WERKZEUGKOPPLUNG SOWIE ZUGEHÖRIGE SYSTEME
RACCORD D'OUTIL À LIBÉRATION RAPIDE AINSI QUE SYSTÈMES ASSOCIÉS

(30) Priority: 25.08.2016 US 201662379344 P
(43) Date of publication of application: 03.07.2019
(73) Proprietor: Board of Regents of the University of Nebraska, Lincoln, Nebraska 68583-0745 (US)
(72) Inventor: FARRITOR, Shane, Lincoln, Nebraska 68526 (US); FREDERICK, Thomas, Lincoln, Nebraska 68505 (US)
(74) Representative: Bradbury, Simon Timothy Nicholas
(86) International application number: PCT/US2017/048688
(87) International publication number: WO 2018/039606

(56) References cited:
- EP-A1- 2 684 528
- EP-A1- 2 815 705
- EP-A1- 2 937 047
- EP-A2- 2 881 046
- US-A1- 2010 286 480
- US-A1- 2011 132 960
- US-A1- 2014 249 474
- US-A1- 2014 373 652
- US-A1- 2016 143 688

## Description

### Field of the Invention

The various embodiments herein relate to coupling mechanisms that provide for quick coupling to and quick release from a medical device tool such as, for example, an end effector. The various coupling mechanism embodiments can be incorporated into or attached to various types of medical devices, including robotic surgical devices and systems.

### Background of the Invention

Many known surgical device systems, including robotic systems, utilize a tool coupler that consists of concentric splines and a quarter-turn system to lock the tool into the front of the device (or an arm thereof). In other words, the coupler requires that the tool be positioned in the coupler on the device and rotated ¼ turn to align the concentric splines and thereby couple or attach the tool to the device. In these known couplers, once the tool is attached to the device, the concentric splines also operate to transfer rotary motion from the device to the tool.

There is a need in the art for an improved end effector tool coupler for use with various types of medical devices.

EP2815705A1 discloses a surgical device adapter for coupling an end effector to a handle assembly for performing endoscopic surgical procedures, for use with removable disposable loading units and/or single use loading units for clamping, cutting and/or stapling tissue. EP2684528A1 discloses an adapter assembly for selectively interconnecting a surgical end effector that is configured to perform a function and a surgical device that is configured to actuate the end effector, the end effector including at least one axially translatable drive member, and the surgical device including at least one rotatable drive shaft. EP2881046A1 and EP2937047A1 disclose adapter assemblies for use with and to electrically and mechanically interconnect electromechanical surgical devices and surgical loading units.

### Brief Summary of the Invention

Discussed herein are various coupling mechanisms, apparatuses, and components for quick-release attachment of various medical tools to various medical devices and systems.

The present invention provides a coupling apparatus and a coupling system as defined in the appended claims.

While multiple embodiments are disclosed, still other embodiments of the present invention will become apparent to those skilled in the art from the following detailed description, which shows and describes illustrative embodiments of the invention. As will be realized, the invention is capable of modifications in various obvious aspects, all without departing from the scope of the present invention as defined in the appended claims. Accordingly, the drawings and detailed description are to be regarded as illustrative in nature and not restrictive.

### Brief Description of the Drawings

FIG. 1A is a side view of a coupling mechanism coupled to a device tool, according to one embodiment.
FIG. 1B is a side view of the coupling mechanism and device tool of FIG. 1A in which the locking mechanism has been depressed, according to one embodiment.
FIG. 1C is a side view of the coupling mechanism and device tool of FIG. 1A in which the device tool is being uncoupled from the coupling mechanism, according to one embodiment.
FIG. 2A is a side view of a graspers end effector, according to one embodiment.
FIG. 2B is a perspective rear view of the graspers end effector of FIG. 2A.
FIG. 3 is a perspective front view of a coupling mechanism, according to another embodiment.
FIG. 4A is a perspective front view of a graspers end effector, according to another embodiment.
FIG. 4B is a side cutaway view of the graspers end effector of FIG. 4A coupled to a coupling mechanism, according to one embodiment.
FIG. 5A is a side cutaway view of a coupling mechanism, according to one embodiment.
FIG. 5B is a side cutaway view of the coupling mechanism of FIG. 5A coupled to a device tool, according to one embodiment.

### Detailed Description

The various systems and devices disclosed herein relate to devices for use in medical procedures and systems. More specifically, various embodiments relate to a quick-change coupling apparatus or component that can be used to releasably couple a tool or end effector to a medical device or a component thereof (such as, for example, an arm of the device). For example, in certain implementations, the medical device is a robotic surgical device with an arm having the coupling mechanism disposed on the arm such that one or more end effectors can be coupled to and detached from the arm via the coupling mechanism.

Rather than the known quarter-turn configuration as discussed above, the implementations disclosed or contemplated herein relate to a self-locking quick release mechanism that includes a spring-loaded coupling component (also referred to herein as an "coupler" or "coupler) (rather than concentric splines) that provides for a compliant passage of actuation forces without requiring any type of alignment step during the process of coupling the tool to the coupling component.

The various systems and devices disclosed herein relate to devices, or components thereof, for use in medical procedures and systems. More specifically, various embodiments relate to various medical devices, including robotic devices and related methods and systems.

It is understood that the various embodiments of robotic devices and related methods and systems disclosed herein can be incorporated into or used with any other known medical devices, systems, and methods. For example, the various embodiments disclosed herein may be incorporated into or used with any of the medical devices and systems disclosed in U.S. Patent 8,968,332 (issued on March 3, 2015 and entitled "Magnetically Coupleable Robotic Devices and Related Methods"), U.S. Patent 8,834,488 (issued on September 16, 2014 and entitled "Magnetically Coupleable Surgical Robotic Devices and Related Methods"), U.S. Patent Application 14/617,232 (filed on February 9, 2015 and entitled "Robotic Surgical Devices and Related Methods"), U.S. Patent 9,579,088 (issued on February 28, 2017 and entitled "Methods, Systems, and Devices for Surgical Visualization and Device Manipulation"), U.S. Patent 8,343,171 (issued on January 1, 2013 and entitled "Methods and Systems of Actuation in Robotic Devices"), U.S. Patent 8,828,024 (issued on September 9, 2014 and entitled "Methods and Systems of Actuation in Robotic Devices"), U.S. Patent Application 14/454,035 (filed August 7, 2014 and entitled "Methods and Systems of Actuation in Robotic Devices"), U.S. Patent Application 12/192,663 (filed August 15, 2008 and entitled Medical Inflation, Attachment, and Delivery Devices and Related Methods"), U.S. Patent Application 15/018,530 (filed February 8, 2016 and entitled "Medical Inflation, Attachment, and Delivery Devices and Related Methods"), U.S. Patent 8,974,440 (issued on March 10, 2015 and entitled "Modular and Cooperative Medical Devices and Related Systems and Methods"), U.S. Patent 8,679,096 (issued on March 25, 2014 and entitled "Multifunctional Operational Component for Robotic Devices"), U.S. Patent 9,179,981 (issued on November 10, 2015 and entitled "Multifunctional Operational Component for Robotic Devices"), U.S. Patent Application 14/936,234 (filed on November 9, 2015 and entitled "Multifunctional Operational Component for Robotic Devices"), U.S. Patent 8,894,633 (issued on November 25, 2014 and entitled "Modular and Cooperative Medical Devices and Related Systems and Methods"), U.S. Patent 8,968,267 (issued on March 3, 2015 and entitled "Methods and Systems for Handling or Delivering Materials for Natural Orifice Surgery"), U.S. Patent 9,060,781 (issued on June 23, 2015 and entitled "Methods, Systems, and Devices Relating to Surgical End Effectors" ), U.S. Patent Application 14/745,487 (filed on June 22, 2015 and entitled "Methods, Systems, and Devices Relating to Surgical End Effectors" ), U.S. Patent 9,089,353 (issued on July 28, 2015 and entitled "Robotic Surgical Devices, Systems, and Related Methods"), U.S. Patent Application 14/800,423 (filed on July 15, 2015 and entitled "Robotic Surgical Devices, Systems, and Related Methods"), U.S. Patent Application 13/573,849 (filed October 9, 2012 and entitled "Robotic Surgical Devices, Systems, and Related Methods"), U.S. Patent Application 13/738,706 (filed January 10, 2013 and entitled "Methods, Systems, and Devices for Surgical Access and Insertion"), U.S. Patent Application 13/833,605 (filed March 15, 2013 and entitled "Robotic Surgical Devices, Systems, and Related Methods"), U.S. Patent Application 14/661,465 (filed March 18, 2015 and entitled "Methods, Systems, and Devices for Surgical Access and Insertion"), U.S. Patent 9,498,292 (issued on November 22, 2016 and entitled "Single Site Robotic Devices and Related Systems and Methods"), U.S. Patent Application 15/357,663 (filed November 21, 2016 and entitled "Single Site Robotic Devices and Related Systems and Methods"), U.S. Patent 9,010,214 (issued on April 21, 2015 and entitled "Local Control Robotic Surgical Devices and Related Methods"), U.S. Patent Application 14/656,109 (filed on March 12, 2015 and entitled "Local Control Robotic Surgical Devices and Related Methods"), U.S. Patent Application 14/208,515 (filed March 13, 2014 and entitled "Methods, Systems, and Devices Relating to Robotic Surgical Devices, End Effectors, and Controllers"), U.S. Patent Application 14/210,934 (filed March 14, 2014 and entitled "Methods, Systems, and Devices Relating to Force Control Surgical Systems), U.S. Patent Application 14/212,686 (filed March 14, 2014 and entitled "Robotic Surgical Devices, Systems, and Related Methods"), U.S. Patent Application 14/334,383 (filed July 17, 2014 and entitled "Robotic Surgical Devices, Systems, and Related Methods"), U.S. Patent Application 14/853,477 (filed September 14, 2015 and entitled "Quick-Release End Effectors and Related Systems and Methods"), U.S. Patent Application 14/938,667 (filed November 11, 2015 and entitled "Robotic Device with Compact Joint Design and Related Systems and Methods"), U.S. Patent Application 15/227,813 (filed August 3, 2016 and entitled "Robotic Surgical Devices, Systems, and Related Methods"), U.S. Patent Application 15/599,231 (filed May 18, 2017 and entitled "Robotic Surgical Devices, Systems, and Related Methods"), U.S. Patent Application 62/381,299 (filed August 30, 2016 and entitled "Robotic Device with Compact Joint Design and an Additional Degree of Freedom and Related Systems and Methods"), U.S. Patent Application 62/425,149 (filed November 22, 2016 and entitled "Improved Gross Positioning Device and Related Systems and Methods"), U.S. Patent Application 62/427,357 (filed November 29, 2016 and entitled "Controller with User Presence Detection and Related Systems and Methods"), U.S. Patent Application 62/433,837 (filed December 14, 2016 and entitled "Releasable Attachment Device for Coupling to Medical Devices and Related Systems and Methods"), and U.S. Patents 7,492,116 (filed on October 31, 2007 and entitled "Robot for Surgical Applications"), 7,772,796 (filed on April 3, 2007 and entitled "Robot for Surgical Applications"), and 8,179,073 (issued May 15, 2011, and entitled "Robotic Devices with Agent Delivery Components and Related Methods"). Certain device and system implementations disclosed in the applications listed above can be positioned within a body cavity of a patient in combination with a support component similar to those disclosed herein. An "in vivo device" as used herein means any device that can be positioned, operated, or controlled at least in part by a user while being positioned within a body cavity of a patient, including any device that is coupled to a support component such as a rod or other such component that is disposed through an opening or orifice of the body cavity, also including any device positioned substantially against or adjacent to a wall of a body cavity of a patient, further including any such device that is internally actuated (having no external source of motive force), and additionally including any device that may be used laparoscopically or endoscopically during a surgical procedure. As used herein, the terms "robot," and "robotic device" shall refer to any device that can perform a task either automatically or in response to a command.

Certain embodiments provide for insertion of the present invention into the cavity while maintaining sufficient insufflation of the cavity. Further embodiments minimize the physical contact of the surgeon or surgical users with the present invention during the insertion process. Other implementations enhance the safety of the insertion process for the patient and the present invention. For example, some embodiments provide visualization of the present invention as it is being inserted into the patient's cavity to ensure that no damaging contact occurs between the system/device and the patient. In addition, certain embodiments allow for minimization of the incision size/length. Further implementations reduce the complexity of the access/insertion procedure and/or the steps required for the procedure. Other embodiments relate to devices that have minimal profiles, minimal size, or are generally minimal in function and appearance to enhance ease of handling and use.

Certain implementations disclosed herein relate to "combination" or "modular" medical devices that can be assembled in a variety of configurations. For purposes of this application, both "combination device" and "modular device" shall mean any medical device having modular or interchangeable components that can be arranged in a variety of different configurations. The modular components and combination devices disclosed herein also include segmented triangular or quadrangular-shaped combination devices. These devices, which are made up of modular components (also referred to herein as "segments") that are connected to create the triangular or quadrangular configuration, can provide leverage and/or stability during use while also providing for substantial payload space within the device that can be used for larger components or more operational components. As with the various combination devices disclosed and discussed above, according to one embodiment these triangular or quadrangular devices can be positioned inside the body cavity of a patient in the same fashion as those devices discussed and disclosed above.

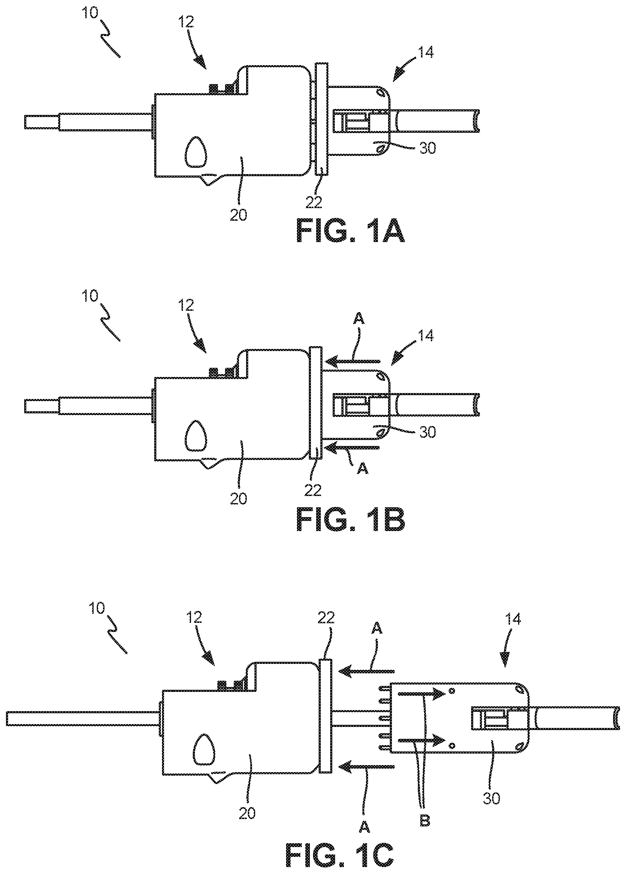

FIGS. 1A-1C depict one embodiment of a self-locking quick release mechanism 10 for coupling a device tool 14 to a coupler 12. It is understood that the coupler 12 is coupled to or integral with a medical device or some component thereof, such that the coupling of a device tool 14 to the coupler 12 results in the device tool 14 being coupled to the medical device. For example, in certain implementations, the coupler 12 is coupled to or integral with a distal end of a robotic arm of the medical device. In more specific implementations, the coupler 12 is coupled to or integral with a distal end of a forearm of a robotic arm of the medical device. The coupler 12 has a coupler body 20 and an actuable locking ring 22. The actuable locking ring 22 can be depressed (or urged proximally toward the coupler body 20) as shown by the arrows A in FIG. 1B to trigger the release of the device tool 14 from the coupler 12.

The removable device tool 14, according to some implementations, is an end effector 14 for coupling to an arm of a medical device via the coupler 12. Alternatively, the end effector 14 is being coupled to a distal end of a forearm of a medical device via the coupler 12. The removable device tool 14 can have any number of different configurations or can be any one of several different types of tools. Regardless of the configuration of the tool 14, it has a tool body 30 that is configured to be positionable in and coupleable with the coupler body 20.

In use, the tool 14 can be removed or uncoupled from the coupler 12 by urging the actuable locking ring 22 proximally toward the coupler body 20 as shown in FIG. 1B, thereby releasing the tool 14 such that it can be urged distally as shown via the arrows B in FIG. 1C and removed from the coupler 12. It is understood that after the tool 14 has been removed, the tool 14 can be re-attached to the coupler 12 - or another tool 14 can be attached thereto - by simply urging the tool 14 proximally into the locking ring 22 such that the tool 14 couples to the coupler 12.

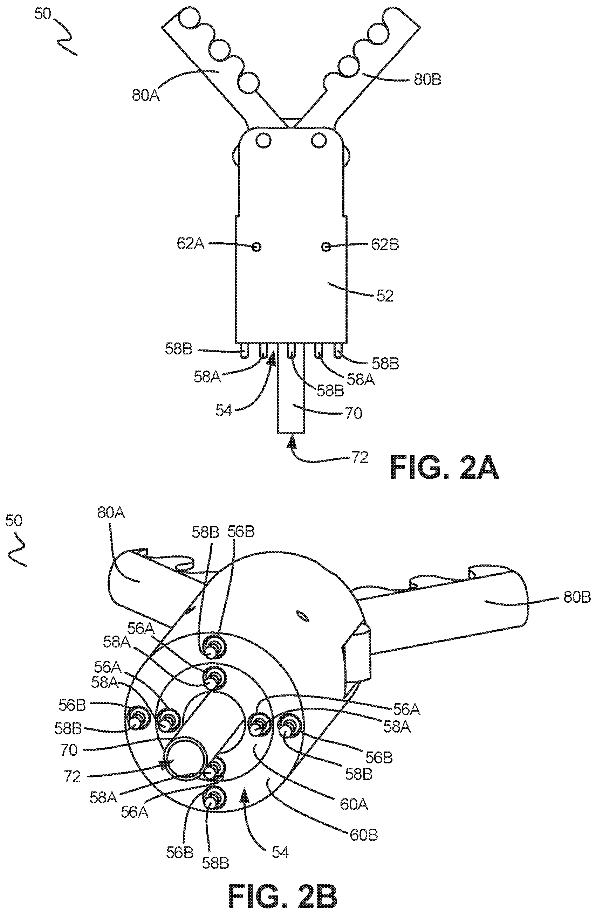

One exemplary tool 50 that is coupleable to a coupler (such as coupler 12 discussed above) is depicted in FIGS. 2A and 2B and has an end effector body 52. As best shown in FIG. 2B, the proximal end of the end effector body 52 has pins (also referred to herein as "tensioned pins" or "spring-loaded pins") 58A, 58B disposed within and extending from the proximal end 54 of the body 52 in their resting state. Each of the pins 58A, 58B is disposed within an opening (also referred to herein as a "pin chamber") 56A, 56B defined in the proximal end 54 such that each pin 58A, 58B can be urged toward the body 52 into its chamber 56A, 56B. That is, each pin 58A, 58B is coupled to a force mechanism (not shown) that has a resting state in which the pin 58A, 58B is extended from the pin chamber 56A, 56B and applies a force to the pin 58A, 58B when the pin 58A, 58B is urged toward the end effector body 52. In one embodiment, the force mechanism is a compression spring (not shown). Alternatively, any known force mechanism that operates as described can be used.

In the exemplary embodiment as shown, the end effector body 52 has eight spring-loaded pins 58A, 58B, with four pins 58A disposed in four pin chambers 56A defined in an inner driven component 60A and four pins 58B disposed in four pin chambers 56B defined in an outer driven component 60B, wherein the two driven components 60A, 60B are concentric or coaxial. That is, the two driven components 60A, 60B are separate components that rotate around the same axis. Alternatively, the body 52 can have a number of pins ranging from one pin to any number of pins that can be disposed in chambers on the proximal end 54 of the body 52. In one specific alternative embodiment, the proximal end 54 has at least four pins disposed in four pin chambers. In a further alternative, the proximal end 54 has at least two pins disposed in two pin chambers.

In this specific implementation, the end effector body 52 also has a central tube 70 disposed therethrough that defines a central lumen 72 within the tube 70. The central tube 70 can be used in several different capacities, thereby making it possible for the tool 50 to be one of several different types of tools. That is, the tube 70 with its central lumen 72 can be used for suction, irrigation, tool delivery, drug delivery, clip application, and/or biopsy collection, and various other known features of various medical device tools or end effectors.

Alternatively, there are tool configurations that don't require a tube 70 with a lumen 72, and thus the body 52 according to certain implementations can have no tube. Without the tube 70, the body 52 can have a smaller diameter. In one specific embodiment in which the body 52 has no tube, the body can have a diameter of around 9.5 mm (3/8 inch), thereby allowing the end effector 50 to fit through a standard laparoscopic port (which has an inner diameter of around 10 mm.

In one implementation, the coaxial driven components 60A, 60B can rotate in relation to each other, thereby adding an additional degree of freedom to the tool 50. In alternative embodiments, the body 52 doesn't have two concentric driven components, but instead the body 52 is a single, unitary component.

In the exemplary embodiment as shown in FIGS. 2A and 2B, the tool 50 is a set of graspers 50. That is, the graspers end effector 50 has two grasper arms 80A, 80B coupled to the end effector body 52.

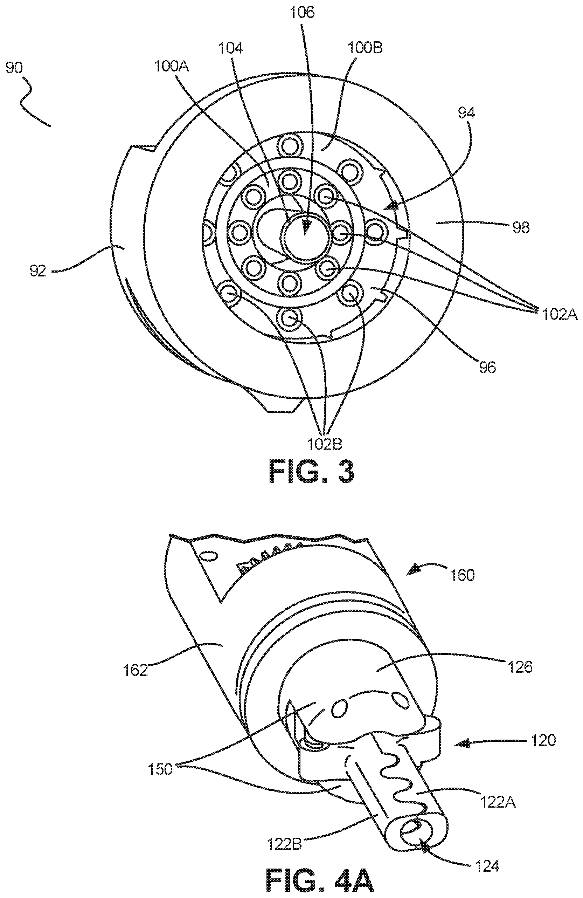

The spring-loaded pins 58A, 58B on the tool 50 as described above are configured to operate in conjunction with a corresponding device coupler (such as the coupler 12 discussed above, for example, or any other coupler embodiment disclosed or contemplated herein) to allow for the coupling of the tool body 50 to the coupler without the need for an alignment step. This non-alignment coupling is best described in relation to the coupler to which the body 50 is coupled. One example of a device coupler 90 is depicted in FIG. 3 according to one implementation, in which the coupler 90 has a coupler body 92, a coupler cavity 94, a coupler drive component 96 disposed within the cavity 94, and an actuable locking ring 98 disposed around the cavity 94. In this specific implementation, the coupler drive component 96 is actually made up of two drive components: a first or inner drive component 100A and a second or outer drive component 100B, wherein the drive components 100A, 100B are coaxial and rotatable in relation to each other. Further, each of the drive components 100A, 100B has pin-receiving openings 102A, 102B defined therein. More specifically, in this particular embodiment, the inner drive component 100A has eight openings 102A and the outer drive component 100B has eight openings 102B. The eight openings 102A defined in the inner drive component 100A are configured to receive the spring-loaded pins of an inner driven component of a proximal end of a coupleable tool (such as the pins 58A of the inner driven component 60A of the tool 50 discussed above, for example), while the eight openings 102B defined in the outer drive component 100B are configured to receive the spring-loaded pins of an outer driven component of a proximal end of a coupleable tool (such as the pins 58B of the outer driven component 60B of the tool 50 discussed above, for example). Alternatively, the coupler drive component 96 doesn't have two concentric drive components and instead has a single, unitary component and thus is configured to couple with the proximal end of a coupleable tool that also has a single, unitary component.

These openings 102A, 102B are defined in a predetermined pattern on the drive component 96 such that the pins 58A, 58B can fit into the openings 102A, 102B. In this embodiment, the inner drive component 100A has twice as many openings 102A as the number of pins 58A on the inner driven component 60A of the tool 50 and the outer drive component 100B has twice as many openings 102B as the number of pins 58B on the outer driven component 60B of the tool 50. As such, the pins 58A, 58B can be positioned in the openings 102A, 102B in two different couplings (in two different sets of the openings 102A, 102B). As such, the fact that there are twice as many openings 102A, 102B as pins 58A, 58B further reduces the coupling time, as will be described in additional detail below.

In addition, this coupler 90 embodiment has a central tube 104 with a lumen 106 that is coupleable to any central tube of the tool to be coupled thereto (such as the tube 70 of the tool 50 described above). Alternatively, the coupler 90 does not have a central tube 104 when the tool to be coupled thereto has no central tube.

In use in which the tool 50 is coupled to the coupler 90, the proximal end 54 of the tool body 52 is inserted into the coupler cavity 90 and urged proximally toward the coupler drive component 96. While it is unlikely, if the pins 58A, 58B happen to be aligned correctly with the openings 102A, 102B without any rotation of either the tool 50 or the coupler 90 in relation to each other, the pins 58A, 58B will be urged into the openings 102A, 102B and disposed therein such that rotation of the inner drive component 100A of the coupler drive component 96 will cause rotation of the inner driven component 60A of the tool 50 and rotation of the outer drive component 100B of the drive component 96 will cause rotation of the outer driven component 60B of the tool 50. In the more likely scenario that the pins 58A, 58B are not aligned correctly with the openings 102A, 102B, the pins 58A, 58B will make contact with the drive component 96 such that the pins 58A, 58B will be urged toward the device body 52 such that the pins 58A, 58B will be urged into their pin chambers 56A, 56B until the proximal end 54 contacts the coupler drive component 96. At this point, the two drive components 100A, 100B of the drive component 96 are rotated in relation to the tool body 52 until the openings 102A, 102B are aligned correctly with the pins 58A, 58B. When the alignment is correct, the force mechanisms (not shown) coupled to each of the pins 58A, 58B will urge the pins proximally toward the coupler body 92, thereby causing the pins 58A, 58B to be positioned in the openings 102A, 102B. Once the pins 58A, 58B are positioned correctly in the openings 102A, 102B, rotation of the inner drive component 100A of the coupler drive component 96 will cause rotation of the inner driven component 60A of the tool 50 and rotation of the outer drive component 100B of the drive component 96 will cause rotation of the outer driven component 60B of the tool 50.

In accordance with one implementation, the coupler 90 having a drive component 96 with openings 102A, 102B makes it easier to sterilize the coupler 90 in comparison to pins (such as pins 58A, 58B), which can be more difficult to sterilize given the additional moving components, relative inaccessibility of some of those components, and related amount of surface area. However, in an alternative embodiment, the coupler (such as coupler 90) could have spring-loaded pins and the tool (such as tool 50) could have openings configured to receive those pins.

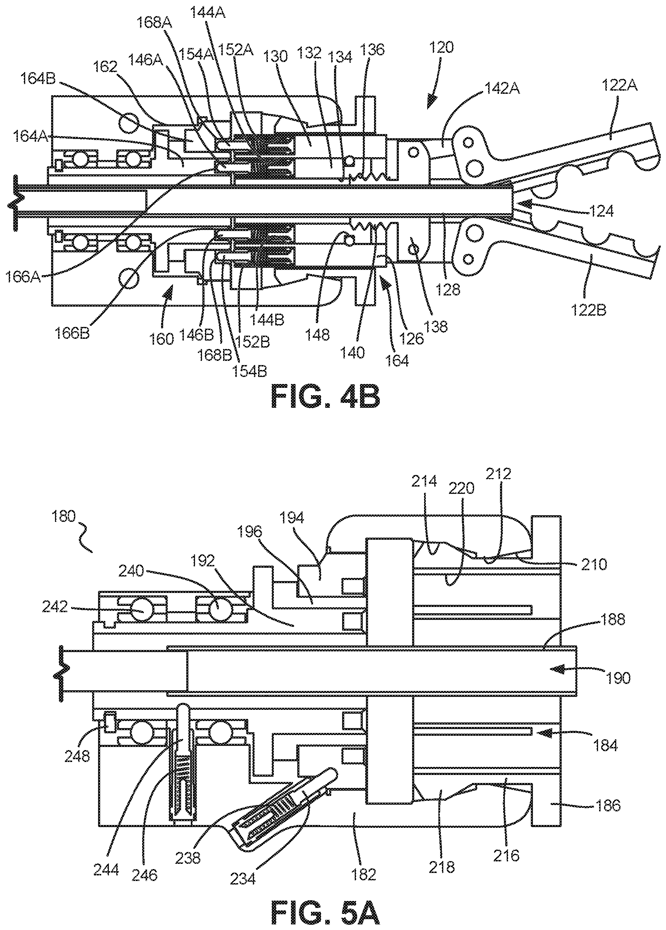

FIGS. 4A and 4B depict another device tool 120 that is a graspers tool 120 with suction and irrigation features and is coupled to a coupler 160. More specifically, the tool body 126 is disposed within the cavity (not shown) of the coupler 160. In this embodiment, the device tool 120 is an end effector 120 and the coupler 160 is coupled to or integral with the arm of a robotic device (not shown). The tool 120 has first and second grasper arms 122A, 122B that are configured to form the distal end of a lumen 124 when the two arms 122A, 122B are in their closed position as best shown in FIG. 4A. As best shown in FIG. 4B, the lumen 124 extends from the grasper arms 122A, 122B to the proximal end of the tool body 126 through a central tube 128. The tube 128 is configured to transport irrigation fluid distally to the distal end of the tool 120 and apply suction proximally toward the proximal end of the body 126 through the lumen 124.

As best shown in FIG. 4B, the tool body 126 is made up of an outer driven component 130 and an inner driven component 132 having an inner lumen 134 with threads 136. The tool 120 also has a push rod 138 that is disposed within the inner lumen 134 and extends distally from the lumen 134. The push rod 138 has external threads 140 that mate with the threads 136 of the inner driven component 132. In addition, the rod 138 is coupled at its distal end to the arm links 142A, 142B (wherein only the arm link 142A is depicted in FIG. 4B) that are coupled to the grasper arms 122A, 122B such that actuation of the push rod 138 causes actuation of the arms 122A, 122B to move between their open and closed configurations. The proximal end of the inner driven component 132 has two pin chambers 144A, 144B defined therein such that each chamber 144A, 144B contains a spring-loaded pin 146A, 146B that is configured to be extendable from the chamber 144A, 144B in the manner discussed above with respect to spring-loaded pins 58A, 58B. While two pin chambers 144A, 144B are depicted, it is understand that the inner driven component 132 can have additional chambers that are not visible in the cross-sectional view depicted in FIG. 4B. As such, the inner driven component 132 can have a similar number of chambers as the inner driven component 60A of the tool body 52 described above and shown in FIG. 2B. In addition, the inner driven component 132 in this embodiment has an external channel 148 defined around an outer surface of the component 132. The channel 148 is configured to receive two cylindrical pins (not shown) that are inserted through openings in the tool body 126 similar to the pins 62A, 62B positioned in the tool body 52 as shown in FIG. 2A. These pins prevent the inner driven component 132 from moving laterally while allowing the component 132 to rotate.

The outer driven component 130 is rotatably disposed around the inner driven component 132 as best shown in FIG. 4B and rotationally coupled to (or integral with) the yoke 150 as best shown in FIG. 4A such that rotation of the outer driven component 130 causes rotation of the yoke 150, thereby rotating the grasper arms 122A, 122B. The proximal end of the outer driven component 130 has two pin chambers 152A, 152B defined therein such that each chamber 152A, 152B contains a spring-loaded pin 154A, 154B that is configured to be extendable from the chamber 152A, 152B in the manner discussed above with respect to spring-loaded pins 58A, 58B. While two pin chambers 152A, 152B (and pins 154A, 154B) are depicted, it is understood that the outer driven component 130 can have additional chambers that are not visible in the cross-sectional view depicted in FIG. 4B. As such, the outer driven component 130 can have a similar number of chambers (and pins) as the outer driven component 60B of the tool body 52 described above and shown in FIG. 2B.

As best shown in FIG. 4B, in accordance with one embodiment, the coupler 160 has a coupler body 162 that contains the coupler drive component 164. In this specific implementation, the coupler drive component 164 is made up of the inner drive component 164A and the outer drive component 164B. The inner drive component 164A as shown has two pin-receiving openings 166A, 166B, each of which is configured to receive a corresponding spring-loaded pin as a result of the coupling action described above. More specifically, as shown in FIG. 4B, pin 146A is disposed in opening 166A and pin 146B is disposed in opening 166B. While two openings 166A, 166B are depicted, it is understood that the inner drive component 164A can have additional openings that are not visible in the cross-sectional view depicted in FIG. 4B. As such, the inner drive component 164A can have a similar number of openings as the inner drive component 100A of the coupler drive component 96 described above and shown in FIG. 3.

Further, the outer drive component 164B as shown has two pin-receiving openings 168A, 168B, each of which is configured to receive a corresponding spring-loaded pin as a result of the coupling action described above. More specifically, as shown in FIG. 4B, pin 154A is disposed in opening 168A and pin 154B is disposed in opening 168B. While two openings 168A, 168B are depicted, it is understood that the outer drive component 164B can have additional openings that are not visible in the cross-sectional view depicted in FIG. 4B. As such, the outer drive component 164B can have a similar number of openings as the outer drive component 100B of the coupler drive component 96 described above and shown in FIG. 3.

In use, the inner drive component 164A of the coupler 160 can be actuated to rotate. With the spring-loaded pins (including pins 146A, 146B) of the tool 120 disposed within the pin-receiving openings 166A, 166B of the inner drive component 164A, the rotation of the inner drive component 164A causes the inner driven component 132 to rotate. Because the internal threads 136 of the inner driven component 132 are mated with the external threads 140 of the push rod 138, the rotation of the inner driven component 132 causes the push rod 138 to move laterally. Because the grasper arm 122A, 122B are coupled to the push rod 138 via the links 142A, 142B (wherein only 142A is depicted in FIG. 4B), the lateral movement of the push rod 138 causes the grasper arms 122A, 122B to move between their open and closed configurations.

Further, the outer drive component 164B can also be actuated to rotate. With the spring-loaded pins (including pins 154A, 154B) of the tool 120 disposed within the pin-receiving openings 168A, 168B of the outer drive component 164B, the rotation of the outer drive component 164B causes the outer driven component 130 to rotate. Because the yoke 150 is coupled to or integral with the distal end of the outer driven component 130 (as best shown in FIG. 4A), the rotation of the outer driven component 130 causes the yoke 150 to rotate. Because the grasper arms 122A, 122B are disposed at least partially within the yoke 150 and are rotationally constrained by the yoke 150, the rotation of the yoke 150 causes the grasper arms 122A, 122B to rotate around the same axis.

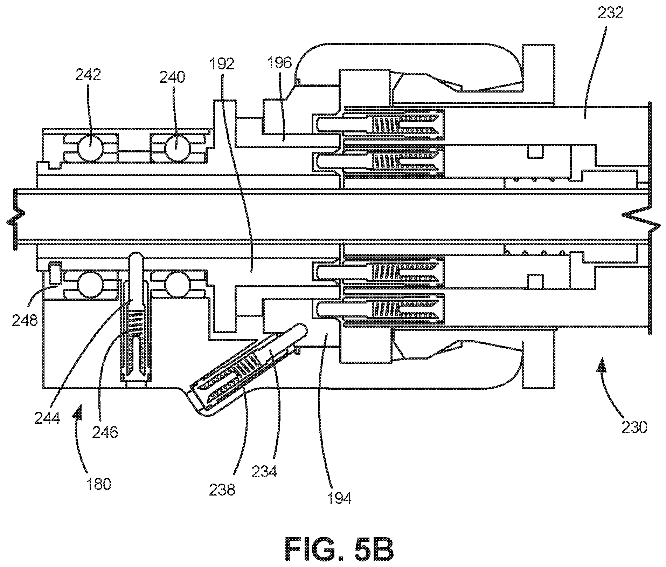

FIGS. 5A and 5B depict another embodiment of a coupler 180 coupled to a tool 230, wherein the coupler 180 and tool 230 are configured such that the tool 230 can have bipolar capabilities as will be described below. FIG. 5A depicts the coupler 180 without the tool 230 coupled thereto, while FIG. 5B depicts the coupler 180 and tool 230 coupled together. In this implementation, the coupler 180 and the tool 230 have components and features substantially similar to those described above and depicted in FIGS. 4A and 4B with respect to the coupler 160 and tool 120, except for those differences described herein.

In this embodiment, the coupler 180 is coupled to or integral with the distal end of a forearm of a robotic surgical device (not shown). Alternatively, the coupler 180 can be coupled to or integral with any medical device to which a tool (such as tool 230) is to be coupled. The coupler 180 has a coupler body 182 that has an actuable locking ring 186 disposed within the coupler cavity 184. Further, the body 182 has a central tube 188 that defines a central lumen 190, an inner drive component 192, an outer drive component 194, and an insulation layer 196 disposed between the inner and outer drive components 192, 194, thereby electrically separating the inner and outer drive components 192, 194 to provide for potential bipolar capabilities.

The actuable locking ring 186 can be used to retain or lock the tool 230 in place in the coupler 180 in the following manner. The cavity 184 in this implementation has a narrow portion (or "wall protrusion") 212 defined in the inner wall 210 of the cavity 184. Further, the inner wall 210 also has a wider portion (or "channel") 214 defined in the inner wall proximal to the wall protrusion 212. The actuable locking ring 186 has a corresponding external ring protrusion (also referred to herein as a "fin") 218 extending from an outer wall 216 of the ring 186. In certain embodiments, as the actuable locking ring 186 is moved laterally within the cavity 184, the position of the ring fin 218 in relation to the inner wall channel 214 and the wall protrusion 212 can directly influence the inner diameter of the ring 186. That is, if the ring 186 is disposed within the cavity 184 such that the fin 218 is disposed in the channel 214, the ring 186 has a relatively larger inner diameter. However, if the ring 186 is moved distally within the cavity 184 such that the fin 218 is moved toward the wall protrusion 212, the fin 218 will be urged radially inward, thereby causing the inner diameter of the ring 186 to become smaller. As such, the interaction between the locking ring 186 and the inner wall of the cavity 184 when the locking ring 186 is moved between a locked and an unlocked position causes the inner diameter of the locking ring 186 to be altered, thereby either increasing or reducing the contact friction between the inner wall 220 of the ring 186 and any tool body (such as tool body 232) disposed therein.

Further, the actuable locking ring 186 can also have coupling blades (not shown) disposed along the inner wall 220 of the ring 186 that are configured to enhance the retention of the tool body within the cavity 184 when the inner wall 220 is in contact with the tool body 232. Alternatively, any component or feature can be used that can help to maintain the physical coupling or frictional retention between the inner wall 220 of the ring 186 and the tool body 232.

In use according to one embodiment as best shown in FIG. 1A-1C in combination with FIGS. 5A and 5B, when the locking ring (such as ring 186) is in the locked position as best shown with locking ring 22 in FIG. 1A, the ring fin 218 is disposed adjacent to and in contact with the wall protrusion 212, thereby resulting in a smaller inner diameter of the ring 186 and thus increased contact between the inner wall 220 of the ring 186 and the tool body (such as tool body 30 or tool body 232) disposed therein. This increased contact, along with any retention feature on the inner wall 220 (such as, for example, the retention blades discussed above), results in the tool body (such as body 30 or body 232) being locked or otherwise retained in the coupler 180 (or coupler 12) by the locking ring 186 (or ring 22). Further, as a result of the configuration of the inner wall 210 of the cavity 184 and the configuration of the ring 186, any distal force applied to the tool body 30, 180 will also urge the ring 186 distally as a result of the contact friction between the body 30, 180 and the ring 186, thereby increasing the contact friction between the ring 186 and the body 30, 180. That is, the configuration of the cavity 184 and ring 186 is such that any distal force applied to the tool body 30, 180 actually increases the strength of the locking mechanism.

When the locking ring (such as ring 186) is urged into the unlocked position as best shown in FIGS. 1B and 1C (with respect to ring 22) and FIGS. 5A and 5B (with respect to ring 186, the ring fin 218 is disposed in the channel 214, thereby resulting in a larger inner diameter of the ring 186 (by comparison with the ring 186 in the locked position) and thus decreased (or no) contact between the inner wall 220 of the ring 186 and the tool body (such as tool body 30 or tool body 232) disposed therein. This reduction or elimination of contact results in the tool body (such as body 30 or body 232) being removable from the coupler 180 (or coupler 12).

In this embodiment as shown in FIGS. 5A and 5B, the outer drive component 194 is supplied with an electrical connection via a first electrical contact (also called a "spring pin") 234 that is configured to maintain contact with the drive component 194 while the component 194 is rotating. That is, the spring pin 234 is positioned in the coupler 180 such that it remains in contact with the drive component 194 even when the drive component 194 is actuated to rotate. Further, the spring pin 234 has a force mechanism 238 - in this case, a compression spring - that urges the spring pin 234 toward the drive component 194, thereby further ensuring that contact is maintained.

The insulation layer 196 is positioned between the inner drive component 192 and the outer drive component 194 such that the insulation layer 196 electrically isolates the two drive components 192, 194 from each other. The electrical isolation results in two independent electrical conduction paths to any tool (such as tool 230) coupled to the coupler 180 for potential bipolar capability.

According to the embodiment depicted, the inner drive component 192 is supported by two bearings 240, 242. Further, the coupler 180 has a second electrical contact (also called a "spring pin") 244 disposed between the two bearings 240, 242 that is in contact with the inner drive component 192. The second spring pin 244 has a force mechanism 246 - in this case, a compression spring - that urges the spring pin 244 toward the drive component 192, thereby further ensuring that contact is maintained. As such, the second spring pin 244 provides the second independent electrical source for the tool (such as tool 230) coupled to the coupler 180. Further, the coupler 180 also has a retaining ring 248 that is positioned in the coupler 180 such that it constrains the inner drive component 192 from translating laterally.

In this implementation, the central tube 188 can be used for suction/irrigation, drug delivery, tool delivery, clip application, and/or other known functions or procedures.

In alternative embodiments, the coupler can provide only one electrical connection (instead of two), thereby eliminating the need for electrical isolation and insulation between components. In further alternatives, the coupler can have three or more electrical connections to provide three or more separate, independent electrical sources for three different uses in the tool (such as tool 230).

The coupler embodiments discussed above have included two drive components (an inner drive component and an outer drive component).

The various coupler embodiments disclosed herein can be utilized to simplify various surgical procedures. For example, in those implementations in which medical device is a robotic surgical device, a quick-change coupler on an arm of the surgical device could allow for exchanging end effectors while the arm of the device is positioned within a cavity of the patient. In one such situation, a separate device having at least one additional end effector positioned thereon is positioned in the patient's cavity and operates in conjunction with the device arm and coupler to effect the exchange of one end effector for another on the arm. Alternatively, a separate external device can be inserted into the patient's cavity through a separate or auxiliary port and/or trocar and operates to remove or un-install the end effector from the arm of the robotic device and retract it from the cavity. The new end effector is then attached to the external tool, the tool is re-inserted into the cavity, and the tool operates in conjunction with the device arm to install or attach the new end effector to the coupler.

## Claims

1. A coupling apparatus (12) for a medical device, the apparatus comprising:
(a) a coupler body (20);
(b) a cavity (94) defined in a distal end of the coupler body, the cavity comprising a distal opening suitable for receiving a tool;
(c) a rotatable drive component (96) disposed within the cavity; and
(d) an actuable locking ring (22) disposed around the cavity, the actuable locking ring suitable for locking a tool within the cavity:
**characterised in that** the rotatable drive component disposed within the cavity (c) further comprises:
(i) an inner drive component (100A) comprising at least two inner pin-receiving openings (102A); and
(ii) an outer drive component (100B) comprising at least two outer pin-receiving openings (102B),
the outer drive component radially enclosing the inner drive component and the drive components (100A,100B) being coaxial and rotatable in relation to each other.

2. The coupling apparatus (12) of claim 1, wherein the coupler body (20) is coupleable to a tool (14), wherein the tool comprises:
(a) a tool body (52) sized and arranged to be positionable within the cavity (94); and
(b) a rotatable driven component (60A) operably coupled to the tool body, the rotatable driven component comprising:
(i) an inner driven component (60A) comprising at least two inner pin chambers (55A) defined in the inner driven component and at least two inner axially tensioned pins (58A) disposed within and extendable from the at least two inner pin chambers; and
(ii) an outer driven component (60A) comprising at least two outer pin chambers (56B) defined in the outer driven component and at least two outer axially tensioned pins (58B) disposed within and extendable from the at least two outer pin chambers;
wherein the inner driven component (60A) is alignable with the inner drive component (100A) such that the at least two inner axially tensioned pins (58A) extend into the at least two inner pin-receiving openings (102A), and
wherein the outer driven component (60B) is alignable with the outer drive component (100B) such that the at least two outer axially tensioned pins (58B) extend into the at least two outer pin-receiving openings (102B).

3. The coupling apparatus (12) of claim 1, further comprising an electrical insulation layer (196) disposed between the inner and outer drive components (194).

4. The coupling apparatus (12) of claim 1, wherein the actuable locking ring (22) is movable proximally toward the coupler body (20) from a non-depressed position in which any tool body disposed within the cavity (94) is locked therein to a depressed position in which any tool body (52) disposed within the cavity is releasable.

5. The coupling apparatus (12) of claim 1, further comprising an elongate tube disposed through a length of the coupler body such that the rotatable drive component (96) is disposed around a distal portion of the elongate tube, the elongate tube comprising a lumen in fluid communication with a distal opening of the elongate tube.

6. A coupling system (10) for a medical device, the system comprising:
(a) the coupling apparatus (12) of claim 1; and
(b) a tool body (14) coupleable with the coupling apparatus (12), wherein the tool body is sized and arranged to be positionable within the cavity (94), the tool body comprising:
(i) a rotatable driven component (60A) operably coupled to the tool body, the rotatable driven component comprising:
(A) at least two pin chambers (56A) defined in the rotatable driven component; and
(B) at least two axially tensioned pins (58A) disposed within and extendable from the at least two pin chambers,
wherein the rotatable driven component is alignable with the rotatable drive component (96)such that the at least two axially tensioned pins extend into the at least two pin-receiving openings.

7. The coupling system (10) of claim 6, further comprising an electrical insulation layer (196) disposed between the inner (192) and outer drive components (194).

8. The coupling system (10) of claim 6, wherein the actuable locking ring (22) is movable proximally toward the coupler body (20) from a non-depressed position in which the tool body (52) disposed within the cavity (94) is locked therein to a depressed position in which the tool body is releasable from the cavity.

9. The coupling system (10) of claim 6, further comprising an elongate tube disposed through a length of the coupler body such that the rotatable drive component is disposed around a distal portion of the elongate tube, the elongate tube comprising a lumen in fluid communication with a distal opening of the elongate tube.

10. The coupling system (10) of claim 6, wherein the rotatable drive component (96) comprises:
(a) an inner drive component (100A), wherein the at least two pin-receiving openings comprise at least two inner pin-receiving openings (102A) defined in the inner drive component; and
(b) an outer drive component (100B), wherein the at least two pin-receiving openings further comprise at least two outer pin-receiving openings (102B) defined in the outer drive component; and
wherein the rotatable driven component comprises:
(a) a rotatable inner driven component (60A) wherein:
(i) the at least two pin chambers comprise at least two inner pin chambers (56A) defined in the rotatable inner driven component, and
(ii) the at least two axially tensioned pins comprise at least two inner axially tensioned pins (58A) disposed within and extendable from the at least two inner pin chambers; and
(b) a rotatable outer driven component (60B) wherein:
(i) the at least two pin chambers further comprise at least two outer pin chambers (56B) defined in the rotatable outer driven component; and
(ii) the at least two axially tensioned pins further comprise at least two outer axially tensioned pins (58B) disposed within and extendable from the at least two outer pin chambers,
wherein the rotatable inner driven component is alignable with the inner drive component such that the at least two inner axially tensioned pins extend into the at least two inner pin-receiving openings, and
wherein the rotatable outer driven component is alignable with the outer drive component such that the at least two outer axially tensioned pins extend into the at least two outer pin-receiving openings.

11. The coupling system (10) of claim 10, further comprising an electrical insulation layer (196) disposed between the inner (192) and outer drive components (194).

## Patentansprüche

1. Kupplungseinrichtung (12) für eine medizinische Vorrichtung, die Einrichtung umfassend:
(a) einen Kupplungskörper (20);
(b) einen Hohlraum (94), der an einem distalen Ende des Kupplungskörpers definiert ist, wobei der Hohlraum eine distale Öffnung umfasst, die zum Aufnehmen eines Werkzeugs geeignet ist;
(c) eine drehbare Antriebskomponente (96), die innerhalb des Hohlraums angeordnet ist; und
(d) einen betätigbaren Verriegelungsring (22), der um den Hohlraum herum angeordnet ist, wobei der betätigbare Verriegelungsring zum Verriegeln eines Werkzeugs innerhalb des Hohlraums geeignet ist:
**dadurch gekennzeichnet, dass** die innerhalb des Hohlraums (c) angeordnete drehbare Antriebskomponente weiter umfasst:
(i) eine innere Antriebskomponente (100A), umfassend mindestens zwei innere Stiftaufnahmeöffnungen (102A); und
(ii) eine äußere Antriebskomponente (100B), umfassend mindestens zwei äußere Stiftaufnahmeöffnungen (102B),
wobei die äußere Antriebskomponente die innere Antriebskomponente radial umschließt, und wobei die Antriebskomponenten (100A, 100B) koaxial und relativ zueinander drehbar sind.

2. Kupplungseinrichtung (12) nach Anspruch 1, wobei der Kupplungskörper (20) mit einem Werkzeug (14) koppelbar ist, das Werkzeug umfassend:
(a) einen Werkzeugkörper (52), der so dimensioniert und angeordnet ist, dass er innerhalb des Hohlraums (94) positionierbar ist; und
(b) eine drehbare angetriebene Komponente (60A), die betriebsfähig mit dem Werkzeugkörper gekoppelt ist, die drehbare angetriebene Komponente umfassend:
(i) eine innere angetriebene Komponente (60A), umfassend die mindestens zwei innere Stiftkammern (55A), die in der inneren angetriebenen Komponente definiert sind, und mindestens zwei innere, axial vorgespannte Stifte (58A), die innerhalb der mindestens zwei inneren Stiftkammern angeordnet sind und aus diesen herausragen können; und
(ii) eine äußere angetriebene Komponente (60A), umfassend mindestens zwei äußere Stiftkammern (56B), die in der äußeren angetriebenen Komponente definiert sind, und mindestens zwei äußere, axial vorgespannte Stifte (58B), die innerhalb der mindestens zwei äußeren Stiftkammern angeordnet sind und aus diesen herausragen können;
wobei die innere angetriebene Komponente (60A) mit der inneren Antriebskomponente (100A) ausrichtbar ist, sodass die mindestens zwei inneren, axial vorgespannten Stifte (58A) in die mindestens zwei inneren Stiftaufnahmeöffnungen (102A) hineinragen, und
wobei die äußere angetriebene Komponente (60B) mit der äußeren Antriebskomponente (100B) ausrichtbar ist, sodass die mindestens zwei äußeren, axial vorgespannten Stifte (58B) in die mindestens zwei äußeren Stiftaufnahmeöffnungen (102B) hineinragen.

3. Kupplungseinrichtung (12) nach Anspruch 1, weiter umfassend eine elektrische Isolationsschicht (196), die zwischen der inneren und der äußeren Antriebskomponente (194) angeordnet ist.

4. Kupplungseinrichtung (12) nach Anspruch 1, wobei der betätigbare Verriegelungsring (22) proximal in Richtung des Kupplungskörpers (20) von einer nicht gedrückten Position, in der ein innerhalb des Hohlraums (94) angeordneter Werkzeugkörper darin verriegelt ist, in eine gedrückte Position beweglich ist, in der ein innerhalb des Hohlraums angeordneter Werkzeugkörper (52) freigegeben werden kann.

5. Kupplungseinrichtung (12) nach Anspruch 1, weiter umfassend ein längliches Rohr, das durch eine Länge des Kupplungskörpers angeordnet ist, sodass die drehbare Antriebskomponente (96) um einen distalen Abschnitt des länglichen Rohrs herum angeordnet ist, wobei das längliche Rohr ein Lumen umfasst, das in Fluidverbindung mit einer distalen Öffnung des länglichen Rohrs steht.

6. Kupplungssystem (10) für eine medizinische Vorrichtung, das System umfassend:
(a) die Kupplungseinrichtung (12) nach Anspruch 1; und
(b) einen Werkzeugkörper (14), der mit der Kupplungseinrichtung (12) koppelbar ist, wobei der Werkzeugkörper so dimensioniert und angeordnet ist, dass er innerhalb des Hohlraums (94) positionierbar ist, der Werkzeugkörper umfassend:
(i) eine drehbare angetriebene Komponente (60A), die betriebsfähig mit dem Werkzeugkörper gekoppelt ist, die drehbare angetriebene Komponente umfassend:
(A) mindestens zwei Stiftkammern (56A), die in der drehbaren angetriebenen Komponente definiert sind; und
(B) mindestens zwei axial vorgespannte Stifte (58A), die innerhalb der mindestens zwei Stiftkammern angeordnet sind und aus diesen herausragen können,
wobei die drehbare angetriebene Komponente mit der drehbaren Antriebskomponente (96) ausrichtbar ist, sodass die mindestens zwei axial vorgespannten Stifte in die mindestens zwei Stiftaufnahmeöffnungen hineinragen.

7. Kupplungssystem (10) nach Anspruch 6, weiter umfassend eine elektrische Isolationsschicht (196), die zwischen der inneren (192) und der äußeren Antriebskomponente (194) angeordnet ist.

8. Kupplungssystem (10) nach Anspruch 6, wobei der betätigbare Verriegelungsring (22) proximal in Richtung des Kupplungskörpers (20) von einer nicht gedrückten Position, in der der innerhalb des Hohlraums (94) angeordnete Werkzeugkörper (52) darin verriegelt ist, in eine gedrückte Position beweglich ist, in der der Werkzeugkörper aus dem Hohlraum freigegeben werden kann.

9. Kupplungssystem (10) nach Anspruch 6, weiter umfassend ein längliches Rohr, das durch eine Länge des Kupplungskörpers angeordnet ist, sodass die drehbare Antriebskomponente um einen distalen Abschnitt des länglichen Rohrs herum angeordnet ist, wobei das längliche Rohr ein Lumen umfasst, das in Fluidverbindung mit einer distalen Öffnung des länglichen Rohrs steht.

10. Kupplungssystem (10) nach Anspruch 6, wobei die drehbare Antriebskomponente (96) umfasst:
(a) eine innere Antriebskomponente (100A), wobei die mindestens zwei Stiftaufnahmeöffnungen mindestens zwei innere Stiftaufnahmeöffnungen (102A) umfassen, die in der inneren Antriebskomponente definiert sind; und
(b) eine äußere Antriebskomponente (100B), wobei die mindestens zwei Stiftaufnahmeöffnungen weiter mindestens zwei äußere Stiftaufnahmeöffnungen (102B) umfassen, die in der äußeren Antriebskomponente definiert sind; und
wobei die drehbare angetriebene Komponente umfasst:
(a) eine drehbare innere angetriebene Komponente (60A), wobei
(i) die mindestens zwei Stiftkammern, mindestens zwei innere Stiftkammern (56A) umfassen, die in der drehbaren inneren angetriebenen Komponente definiert sind, und
(ii) die mindestens zwei axial vorgespannten Stifte mindestens zwei innere, axial vorgespannten Stifte (58A) umfassen, die innerhalb der mindestens zwei inneren Stiftkammern angeordnet sind und aus diesen herausragen können; und
(b) eine drehbare äußere angetriebene Komponente (60B), wobei:
(i) die mindestens zwei Stiftkammern weiter mindestens zwei äußere Stiftkammern (56B) umfassen, die in der drehbaren äußeren angetriebenen Komponente definiert sind; und
(ii) die mindestens zwei axial vorgespannten Stifte weiter mindestens zwei äußere, axial vorgespannten Stifte (58B) umfassen, die innerhalb der mindestens zwei äußeren Stiftkammern angeordnet sind und aus diesen herausragen können,
wobei die drehbare innere angetriebene Komponente mit der inneren Antriebskomponente ausrichtbar ist, sodass die mindestens zwei inneren, axial vorgespannten Stifte in die mindestens zwei inneren Stiftaufnahmeöffnungen hineinragen, und wobei die drehbare äußere angetriebene Komponente mit der äußeren Antriebskomponente ausrichtbar ist, sodass die mindestens zwei äußeren, axial vorgespannten Stifte in die mindestens zwei äußeren Stiftaufnahmeöffnungen hineinragen.

11. Kupplungssystem (10) nach Anspruch 10, weiter umfassend eine elektrische Isolationsschicht (196), die zwischen der inneren (192) und der äußeren Antriebskomponente (194) angeordnet ist.

## Revendications

1. Appareil (12) de couplage pour un dispositif médical, l'appareil comprenant :
(a) un corps (20) de coupleur ;
(b) une cavité (94) définie dans une extrémité distale du corps de coupleur, la cavité comprenant une ouverture distale appropriée pour recevoir un outil ;
(c) un composant d'entraînement rotatif (96) disposé à l'intérieur de la cavité ; et
(d) une bague de verrouillage actionnable (22) disposée autour de la cavité, la bague de verrouillage actionnable étant appropriée pour verrouiller un outil à l'intérieur de la cavité :
**caractérisé en ce que** le composant d'entraînement rotatif disposé à l'intérieur de la cavité (c) comprend en outre :
(i) un composant d'entraînement interne (100A) comprenant au moins deux ouvertures internes (102A) de réception de goupille ; et
(ii) un composant d'entraînement externe (100B) comprenant au moins deux ouvertures externes (102B) de réception de goupille,
le composant d'entraînement externe entourant radialement le composant d'entraînement interne et les composants d'entraînement (100A, 100B) étant coaxiaux et rotatifs l'un par rapport à l'autre.

2. Appareil (12) de couplage selon la revendication 1, dans lequel le corps (20) de coupleur peut être couplé à un outil (14), dans lequel l'outil comprend :
(a) un corps (52) d'outil dimensionné et agencé pour pouvoir être positionné à l'intérieur de la cavité (94) ; et
(b) un composant entraîné rotatif (60A) couplé fonctionnellement au corps d'outil, le composant entraîné rotatif comprenant :
(i) un composant entraîné interne (60A) comprenant au moins deux chambres internes (55A) de goupille définies dans le composant entraîné interne et au moins deux goupilles internes (58A) sollicitées axialement en traction disposées à l'intérieur des au moins deux chambres internes de goupille et extensibles à partir de celles-ci ; et
(ii) un composant entraîné externe (60A) comprenant au moins deux chambres externes (56B) de goupille définies dans le composant entraîné externe et au moins deux goupilles externes (58B) sollicitées axialement en traction disposées à l'intérieur des au moins deux chambres externes de goupille et extensibles à partir de celles-ci ;
dans lequel le composant entraîné interne (60A) peut être aligné avec le composant d'entraînement interne (100A) de sorte que les au moins deux goupilles internes (58A) sollicitées axialement en traction s'étendent dans les au moins deux ouvertures internes (102A) de réception de goupille, et
dans lequel le composant entraîné externe (60B) peut être aligné avec le composant d'entraînement externe (100B) de sorte que les au moins deux goupilles externes (58B) sollicitées axialement en traction s'étendent dans les au moins deux ouvertures externes (102B) de réception de goupille.

3. Appareil (12) de couplage selon la revendication 1, comprenant en outre une couche d'isolation électrique (196) disposée entre les composants d'entraînement interne et externe (194).

4. Appareil (12) de couplage selon la revendication 1, dans lequel la bague de verrouillage actionnable (22) peut être déplacée de manière proximale vers le corps (20) de coupleur depuis une position non enfoncée dans laquelle tout corps d'outil disposé à l'intérieur de la cavité (94) est verrouillé dans celle-ci vers une position enfoncée dans laquelle tout corps (52) d'outil disposé à l'intérieur de la cavité peut être libéré.

5. Appareil (12) de couplage selon la revendication 1, comprenant en outre un tube allongé disposé sur toute une longueur du corps de coupleur de telle sorte que le composant d'entraînement rotatif (96) soit disposé autour d'une portion distale du tube allongé, le tube allongé comprenant une lumière en communication fluidique avec une ouverture distale du tube allongé.

6. Système (10) de couplage pour un dispositif médical, le système comprenant :
(a) l'appareil (12) de couplage selon la revendication 1 ; et
(b) un corps (14) d'outil pouvant être couplé à l'appareil (12) de couplage, dans lequel le corps d'outil est dimensionné et agencé pour pouvoir être positionné à l'intérieur de la cavité (94), le corps d'outil comprenant :
(i) un composant entraîné rotatif (60A) couplé fonctionnellement au corps d'outil, le composant entraîné rotatif comprenant :
(A) au moins deux chambres (56A) de goupille définies dans le composant entraîné rotatif ; et
(B) au moins deux goupilles (58A) sollicitées axialement en traction disposées à l'intérieur des au moins deux chambres de goupille et extensibles à partir de celles-ci,
dans lequel le composant entraîné rotatif peut être aligné avec le composant d'entraînement rotatif (96) de sorte que les au moins deux goupilles sollicitées axialement en traction s'étendent dans les au moins deux ouvertures de réception de goupille.

7. Système (10) de couplage selon la revendication 6, comprenant en outre une couche d'isolation électrique (196) disposée entre les composants d'entraînement interne (192) et externe (194).

8. Système (10) de couplage selon la revendication 6, dans lequel la bague de verrouillage actionnable (22) peut être déplacée de manière proximale vers le corps (20) de coupleur depuis une position non enfoncée dans laquelle le corps d'outil (52) disposé à l'intérieur de la cavité (94) est verrouillé dans celle-ci vers une position enfoncée dans laquelle le corps d'outil peut être libéré de la cavité.

9. Système (10) de couplage selon la revendication 6, comprenant en outre un tube allongé disposé sur toute une longueur du corps de coupleur de telle sorte que le composant d'entraînement rotatif soit disposé autour d'une portion distale du tube allongé, le tube allongé comprenant une lumière en communication fluidique avec une ouverture distale du tube allongé.

10. Système (10) de couplage selon la revendication 6, dans lequel le composant d'entraînement rotatif (96) comprend :
(a) un composant d'entraînement interne (100A), dans lequel les au moins deux ouvertures de réception de goupille comprennent au moins deux ouvertures internes (102A) de réception de goupille définies dans le composant d'entraînement interne ; et
(b) un composant d'entraînement externe (100B), dans lequel les au moins deux ouvertures de réception de goupille comprennent en outre au moins deux ouvertures externes (102B) de réception de goupille définies dans le composant d'entraînement externe ; et
dans lequel le composant entraîné rotatif comprend :
(a) un composant entraîné interne rotatif (60A) dans lequel :
(i) les au moins deux chambres de goupille comprennent au moins deux chambres internes (56A) de goupille définies dans le composant entraîné interne rotatif, et
(ii) les au moins deux goupilles sollicitées axialement en traction comprennent au moins deux goupilles internes (58A) sollicitées axialement en traction disposées à l'intérieur des au moins deux chambres internes de goupille et extensibles à partir de celles-ci ; et
(b) un composant entraîné externe rotatif (60B) dans lequel :
(i) les au moins deux chambres de goupille comprennent en outre au moins deux chambres externes (56B) de goupille définies dans le composant entraîné externe rotatif ; et
(ii) les au moins deux goupilles sollicitées axialement en traction comprennent en outre au moins deux goupilles externes (58B) sollicitées axialement en traction disposées à l'intérieur des au moins deux chambres externes de goupille et extensibles à partir de celles-ci,
dans lequel le composant entraîné interne rotatif peut être aligné avec le composant d'entraînement interne de telle sorte que les au moins deux goupilles internes sollicitées axialement en traction s'étendent dans les au moins deux ouvertures internes de réception de goupille, et dans lequel le composant entraîné externe rotatif peut être aligné avec le composant d'entraînement externe de telle sorte que les au moins deux goupilles externes sollicitées axialement en traction s'étendent dans les au moins deux ouvertures externes de réception de goupille.

11. Système (10) de couplage selon la revendication 10, comprenant en outre une couche d'isolation électrique (196) disposée entre les composants d'entraînement interne (192) et externe (194).
